(19) Europäisches Patentamt
European Patent Office
Office européen des brevets

(11) Veröffentlichungsnummer : **0 367 771 B1**

(12) **EUROPÄISCHE PATENTSCHRIFT**

(45) Veröffentlichungstag der Patentschrift : **02.09.92 Patentblatt 92/36**

(21) Anmeldenummer : **88905203.1**

(22) Anmeldetag : **14.06.88**

(86) Internationale Anmeldenummer : **PCT/EP88/00530**

(87) Internationale Veröffentlichungsnummer : **WO 88/10251 29.12.88 Gazette 88/28**

(51) Int. Cl.$^5$ : **C07C 381/00,** C09K 19/30, C09K 19/20, C09K 19/32, C09K 19/18, C09K 19/14

# (54) ARYLSCHWEFELPENTAFLUORIDE.

(30) Priorität : **27.06.87 DE 3721268**

(43) Veröffentlichungstag der Anmeldung : **16.05.90 Patentblatt 90/20**

(45) Bekanntmachung des Hinweises auf die Patenterteilung : **02.09.92 Patentblatt 92/36**

(84) Benannte Vertragsstaaten : **DE GB**

(56) Entgegenhaltungen : **DE-A- 3 333 168**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **REIFFENRATH, Volker**
**Jahnstr. 18**
**W-6101 Rossdorf (DE)**
Erfinder : **EIDENSCHINK, Rudolf**
**Möhnstr.6**
**W-6500 Mainz (DE)**
Erfinder : **WEBER, Georg**
**Wilhelm-Leuschner-Str. 38**
**W-6106 Erzhausen (DE)**

EP 0 367 771 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue Arylschwefelpentafluoride der Formel I,

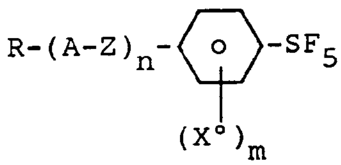

I

worin

R H, Y oder ein unsubstituierter oder durch mindestens eine Gruppe Y substituierter Alkyl- oder Alkenylrest mit jeweils 1-15 C-Atomen, worin auch eine oder mehrere nicht benachbarte $CH_2$-Gruppen durch O- und/oder S-Atome und/oder durch -E-, -CO-, -O-CO-, -CO-O-, -O-CO-O-, -S-CO- und/oder -CO-S-Gruppen ersetzt sein können,

E C≡C,

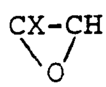 oder 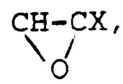

X° jeweils unabhängig voneinander Y oder $CH_3$,

X jeweils unabhängig voneinander X° oder H,

Y CN, $N_3$, NCO, NCS, Fluor oder Chlor

A jeweils unabhängig voneinander

a) 1,4-Cyclohexylenrest,
worin eine oder zwei nicht benachbarte $CH_2$- Gruppen durch O-Atome ersetzt sein können,

b) 1,4-Phenylenrest,
worin ein oder mehrere CH-Gruppen durch N ersetzt sein können,

c) Rest aus der Gruppe 1,4-Cyclohexenylen, Piperidin-1,4-diyl, Bicyclo(2,2,2)octylen, Naphthalin-2,6-diyl, Decahydronaphthalin-2,6-diyl oder 1,2,3,4-Tetrahydronaphthalin, wobei die Reste a) und b) ein- oder zweifach durch X° substituiert sein können,

Z jeweils unabhängig voneinander -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -CH=N-, -N=CH-, -NO=N-, -N=NO- oder eine Einfachbindung,

m 0, 1 oder 2, und

n 1, 2 oder 3 bedeutet.

Der Einfachheit halber bedeuten in folgendem Phe eine 1,4-Phenylengruppe, Cy eine trans-1,4-Cyclohexylengruppe, Che eine Cyclohexenylengruppe, Bi eine Bicyclo(2,2,2)-octylengruppe, Pyd eine Pyridin-2,5-diylgruppe, Pyr eine Pyrimidin-2,5-diylgruppe, Pip eine Piperidin-1,4-diylgruppe, Dit eine 1,3-Dithian-2,5-diylgruppe, Dio eine 1,3-Dioxan-2,5-diylgruppe, wobei diese Gruppen unsubstituiert oder substituiert sein können.

Phe X bedeutet eine Gruppe der Formel

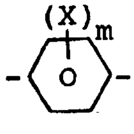

wobei X vorzugsweise im folgenden Chlor oder Fluor bedeutet.

Aus der DE-OS 33 33 168 sind ähnliche Verbindungen bekannt, die jedoch anstelle einer $-SF_5$-Gruppe eine $-S(O)_p-R^2$-Gruppe aufweisen, wobei p 1 oder 2 ist und $R^2$ Alkyl, worin eine oder zwei $CH_2$-Gruppen durch O-Atome ersetzt sein können, bedeutet. Die Ersetzung der Sulfinyl- bzw. Sulfonylgruppe ist für den Fachmann nicht naheliegend, da es keinen Hinweis auf flüssigkristalline Verbindungen mit $SF_5$ als Substituent gab und deren positive Eigenschaften nicht vorhersehbar waren.

Die Verbindungen der Formel I können wie ähnliche Verbindungen als Komponente flüssigkristalliner Phasen verwendet werden, insbesondere für Displays, die auf dem Prinzip der verdrillten Zelle, dem Guest-Host-Effekt, dem Effekt der Deformation aufgerichteter Phasen, dem Effekt der dynamischen Streuung oder dem

2-Frequenzverfahren beruhen.

Die Verbindungen der Formel I eignen sich auch als Komponenten für Phasen zur Verwendung in SBE-, STN- bzw. OMI-Display.

Der Erfindung lag die Aufgabe zugrunde, eine stabile flüssigkristalline oder mesogene Verbindung aufzuzeigen, die als Komponente flüssigkristalliner Phasen geeignet ist.

Es wurde gefunden, daß die Arylschwefelpentafluoride der Formel I vorzüglich als Komponenten flüssigkristalliner Phasen geeignet sind. Insbesondere verfügen sie über vergleichsweise niedere Viskositäten und weisen keine oder nur eine geringe Tendenz zur Bildung molekularer Assoziate auf bei gleichzeitig ausgeprägter positiver dielektrischer Anisotropie. Mit ihrer Hilfe lassen sich stabile flüssigkristalline Phasen mit breitem Mesophasenbereich vorteilhaften Werten für die optische und dielektrische Anisotropie und günstigen elastischen Eigenschaften erhalten.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/ oder dessen Viskosität zu erniedrigen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Arylschwefelpentafluoride der Formel I sowie ein Verfahren zu ihrer Herstellung, dadurch gekennzeichnet, daß man p-Lithium- oder Brommagnesiumarylschwefelpentafluoride mit entsprechenden Elektrophilen umsetzt,

oder daß man entsprechende 1-Alkine mit p-Bromarylschwefelpentafluoriden unter Übergangsmetall-Katalyse koppelt,

oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man zur Herstellung von Estern der Formel I (worin Z -CO-O- oder -O-CO- bedeutet und/oder R eine Carboxylgruppe enthält) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Ethern der Formel I (worin R eine Alkoxygruppe bedeutet und/oder Z eine -$OCH_2$- oder -$CH_2O$-Gruppe ist) eine entsprechende Hydroxyverbindung verethert.

Weiterhin ist Gegenstand der Erfindung die Verwendung der Verbindungen der Formel I als Komponenten flüssigkristalliner Phasen. Gegenstand der Erfindung sind weiterhin flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Die Verbindungen der Formel I umfassen dementsprechend Verbindungen der Teilformeln Ia bis Id (mit zwei Ringen).

R-A-Phe-$SF_5$ Ia

R-A-PheX-$SF_5$ Ib

R-A-Z-Phe-$SF_5$ Ic

R-A-Z-PheX-$SF_5$ Id

Ie bis Il (mit 3 Ringen)

R-A-A-Phe-$SF_5$ Ie

R-A-A-PheX-$SF_5$ If

R-A-A-Z-Phe-$SF_5$ Ig

R-A-A-Z-PheX-$SF_5$ Ih

R-A-Z-A-Phe-$SF_5$ Ii

R-A-Z-A-PheX-$SF_5$ Ij

R-A-Z-A-Z-Phe-$SF_5$ Ik

R-A-Z-A-Z-PheX-$SF_5$ Il

und Im bis Izb (mit 4 Ringen)

R-A-A-A-Phe-$SF_5$ Im

R-A-A-A-PheX-$SF_5$ In

R-A-A-A-Z-Phe-$SF_5$ Io
R-A-A-A-Z-PheX-$SF_5$ Ip
R-A-A-Z-A-Phe-$SF_5$ Iq
R-A-A-Z-A-PheX-$SF_5$ Ir
R-A-Z-A-A-Phe-$SF_5$ Is
R-A-Z-A-A-PheX-$SF_5$ It
R-A-A-Z-A-Z-Phe-$SF_5$ Iu
R-A-A-Z-A-Z-PheX-$SF_5$ Iv
R-A-Z-A-A-Z-Phe-$SF_5$ Iw
R-A-Z-A-A-Z-PheX-$SF_5$ Ix
R-A-Z-A-Z-A-Phe-$SF_5$ Iy
R-A-Z-A-Z-A-PheX-$SF_5$ Iz
R-A-Z-A-Z-A-Z-Phe-$SF_5$ Iza
R-A-Z-A-Z-A-Z-Phe-$SF_5$ Izb

Darunter sind diejenigen der Teilformeln Ia, Id, Ie, Ig besonders bevorzugt.

In den Verbindungen der vor- und nachstehenden Formeln bedeutet R vorzugsweise Alkyl, ferner Alkoxy. Weitere bevorzugte Bedeutungen sind Oxaalkyl, insbesondere 2-Oxa-alkyl, und Alkenyl.

Die Gruppen A, die gleich oder verschieden sein können, sind bevorzugt Cy, Phe, PheX, Pyd oder Pyr; insbesondere bebevorzugt sind Cy oder Phe; bevorzugt enthalten die Verbindungen der Formel I nicht mehr als einen der Reste Bi, Pyd oder Pyr.

In den vor- und nachstehenden Verbindungen der Formel I und allen Teilformeln von I bedeutet PheX bevorzugt eine 1,4-Phenylengruppe, die ein- oder zweifach mit Fluor- oder Chloratomen oder Methyl- oder Cyanogruppen substituiert ist. Insbesondere bevorzugt sind 2-(bzw. 3-)Fluor-1,4-phenylen- und 2,3-Difluor-1,4-phenylengruppen.

n ist vorzugsweise 1 oder 2, insbesondere vorzugsweise 1.

Die Gruppen Z, die gleich oder verschieden sein können, sind bevorzugt Einfachbindungen, in zweiter Linie bevorzugt -CO-O-, -O-CO-, -C≡C- oder -$CH_2CH_2$-Gruppen. Besonders bevorzugt sind Verbindungen der Formel I, worin alle Gruppen Z Einfachbindungen sind oder eine Gruppe Z (vorzugsweise die mit PheX-$SF_5$ verknüpfte Gruppe Z) -CO-O-, -O-CO- oder -$CH_2CH_2$- bedeutet.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2-oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept- 6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine $CH_2$-Gruppe durch -O-CO- oder -CO-O- ersetzt ist, so ist er vorzugsweise geradkettig und hat 2 bis 6 C-Atome. Er bedeutet demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkenylrest bedeutet, in dem eine der C=C-Doppelbindung benachbarte $CH_2$-Gruppe durch -CO- oder -CO-O- oder -O-CO- ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als komponenten für ferroelektrische Materialien.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr $CH_2$-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxynonyl, 10,10-Bis-carboxy-decyl, Bis-(methoxycarbonyl)methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)propyl, 4,4-Bis-(ethoxycarbonyl)-butyl, 5,5-Bis-(ethoxycarbonyl)-pentyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Falls die Verbindungen der Formel I ein asymmetrisches C-Atom enthalten, umfaßt die Formel I Racemate und auch optisch aktive Enantiomere und Enantiomerengemische.

Unter den Verbindungen der Formel I und allen vorstehenden und nachfolgenden Teilformeln von I sind diejenigen bevorzugt, in denen mindestens einer der darin enthaltenen Reste eine der angegebenen bevorzugten Bedeutungen hat.

Dementsprechend umfassen bevorzugte Verbindungen der Teilformel Ia solche der Teilformeln Iaa bis Iaj:

R-Phe-Phe-$SF_5$ Iaa
R-PheX-Phe-$SF_5$ Iab
R-Cy-Phe-$SF_5$ Iac
R-Che-Phe-$SF_5$ Iad
R-Bi-Phe-$SF_5$ Iae
R-Pyd-Phe-$SF_5$ Iaf
R-Pip-Phe-$SF_5$ Iag
R-Dio-Phe-$SF_5$ Iah
R-Dit-Phe-$SF_5$ Iai
R-Pyr-Phe-$SF_5$ Iaj

Von den Verbindungen der Teilformeln Ib-II sind diejenigen der Teilformeln I1 bis I48 besonders bevorzugt:

R-Phe-PheX-$SF_5$ I1
R-Cy-PheX-$SF_5$ I2
R-Bi-PheX-$SF_5$ I3
R-Pyr-PheX-$SF_5$ I4
R-Pyd-PheX-$SF_5$ I5
R-Phe-COO-Phe-$SF_5$ I6
R-Cy-COO-Phe-$SF_5$ I7
R-Bi-COO-Phe-$SF_5$ I8
R-Phe-COO-PheX-$SF_5$ I9
R-Cy-COO-PheX-$SF_5$ I10
R-Bi-COO-PheX-$SF_5$ I11
R-Phe-C≡C-Phe-$SF_5$ I12
R-Cy-C≡C-Phe-$SF_5$ I13
R-Cy-C≡C-PheX-$SF_5$ I14
R-Phe-C≡C-PheX-$SF_5$ I15
R-Bi-$CH_2CH_2$-Phe-$SF_5$ I16
R-Cy-$CH_2CH_2$-PheX-$SF_5$ I17

R-Bi-$CH_2CH_2$-PheX-$SF_5$ I18
R-PheX-COO-Phe-$SF_5$ I19
R-Dio-PheX-$SF_5$ I20
R-Cy-$CH_2CH_2$-Phe-$SF_5$ I21
R-Cy-$CH_2$-O-Phe-$SF_5$ I22
R-Cy-Cy-Phe-$SF_5$ I23
R-Cy-Cy-PheX-$SF_5$ I24
R-Cy-Bi-Phe-$SF_5$ I25
R-Cy-Che-Phe-$SF_5$ I26
R-Dio-Cy-Phe-$SF_5$ I27
R-Cy-Phe-Phe-$SF_5$ I28
R-Cy-Phe-PheX-$SF_5$ I29
R-Phe-Phe-Phe-$SF_5$ I30
R-Phe-Phe-PheX-$SF_5$ I31
R-Cy-Cy-$CO_2$-Phe-$SF_5$ I32
R-Dit-Cy-Phe-$SF_5$ I33
R-Cy-Cy-$CO_2$-PheX-$SF_5$ I34
R-Bi-Phe-OCO-Phe-$SF_5$ I35
R-Cy-Phe-$CO_2$-PheX-$SF_5$ I36
R-Dio-Phe-$CO_2$-PheX-$SF_5$ I37
R-Cy-Phe-$CO_2$-Phe-$SF_5$ I38
R-Dit-Phe-$CO_2$-Phe-$SF_5$ I39
R-Cy-Phe-O-CO-Phe-$SF_5$ I40
R-Pym-Phe-O-CO-Phe-$SF_5$ I41
R-Cy-Phe-C≡C-Phe-$SF_5$ I42
R-Cy-Phe-$CH_2CH_2$-Phe-$SF_5$ I43
R-Py-Phe-C≡C-Phe-$SF_5$ I44
R-Dio-Phe-C≡C-Phe-$SF_5$ I45
R-Phe-Che-Phe-$SF_5$ I46
R-Cy-Che-PheX-$SF_5$ I47
R-Bi-Che-Phe-$SF_5$ I48

Bevorzugte Verbindungen der Teilformeln Im bis Izb sind solche der Teilformeln I49 bis I59:

R-Cy-Phe-Cy-Phe-$SF_5$ I49
R-Cy-Cy-Phe-Phe-$SF_5$ I50
R-Cy-PheX-Phe-COO-Phe-$SF_5$ I51
R-Cy-Phe-Phe-Phe-$SF_5$ I52
R-Cy-Phe-Phe-C≡C-Phe-$SF_5$ I53
R-Phe-Phe-Cy-$CO_2$-Phe-$SF_5$ I54
R-Phe-Phe-Che-$CO_2$-Phe-$SF_5$ I55
R-Phe-Phe-OCO-Che-Phe-$SF_5$ I56
R-Phe-Phe-OCO-Cy-Phe-$SF_5$ I57
R-Cy-Phe-Phe-O-CO-Phe-$SF_5$ I58
R-Cy-Phe-Phe-O-$CH_2$-Phe-$SF_5$ I59

Die Verbindungen der Formel I werden nach an sich bekannten Methoden, wie sie in der Literatur (z.B. in den Standardwerken wie Houben Weyl, Methoden der Organischen Chemie, Georg-Thieme-Verlag, Stuttgart) beschrieben sind aus 4-substituierten Phenylschwefelpentafluoriden, die man z.B nach der Literatur (W.A. Sheppard, J. Am. Chem. Soc. 84, 3064, 1962) erhält, hergestellt.

Dabei geht man von Di(p-Nitrophenyl)disulfid aus, aus dem man durch Umsetzen mit Silberdifluorid p-Nitrophenylschwefelpentafluorid erhält, das wiederum als Ausgangsverbindung vieler 4-substituierter Phenylschwefelpentafluoride dienen kann.

Die Ausgangsstoffe können gewünschtenfalls auch in situ gebildet werden, derart, daß man sie aus dem Reäktionsgemisch nicht isoliert, sondern sofort weiter zu den Verbindungen der Formel I umsetzt.

Ester der Formel I (R = Alkyl, worin eine oder zwei $CH_2$-Gruppen durch -O-CO- und/oder -CO-O-Gruppen ersetzt sind und/oder Z = -CO-O- oder -O-CO- bedeutet) können auch durch Veresterung entsprechender Carbonsäuren (oder ihren reaktionsfähigen Derivaten) mit Alkoholen bzw. Phenolen (oder ihrer reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4

C-Atomen in der Alkylgruppe.

Als reaktionsfähige Derivate der genannten Alkohole bzw Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF, N,N-Dimethylpropylenharnstoff oder Phosphorsäurehexamethyltriamid, Kohlenwasserstoffe wie Benzol, Toluol oder Xylol, Halogenkohlenwasserstoffe wie Dichlormethan, Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin, als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin,Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und mit einem Säureanhydrid oder insbesondere Säurechlorid umsetzt.

Eine bevorzugte Ausführungsform der Veresterung zur Darstellung der Ester der Formel I ist die Umsetzung der entsprechenden Carbonsäure mit dem entsprechenden Alkohol oder Phenol unter der wasserentziehenden Wirkung von Carbodiimiden, insbesondere von Dicyclohexylcarbodiimid, in einem inerten Lösungsmittel, gegebenenfalls unter katalytischem Einsatz von 4-N,N-Dimethylaminopyridin zur Reaktionsbeschleunigung.

Ether der Formel I (worin R eine Alkylgruppe bedeutet, worin eine oder zwei $CH_2$-Gruppen durch O-Atome ersetzt sind, und/oder worin $Z^1$ und/oder $Z^2$ eine $-OCH_2-$ oder eine $-CH_2O-$Gruppe ist) sind durch Veretherung entsprechender Phenole, erhältich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonät oder Dialkylsufonat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen 20° und 100°.

Zur Herstellung von lateral substituierten Fluor- oder Chlor-Verbindungen der Formel I (worin A Aromaten bedeuten, R die übliche Bedeutung hat) können entsprechende Anilinderivate mit Natriumnitrit und entweder mit Tetrafluorborsäure (zur Einführung eines F-Atoms) oder mit Kupfer-(I)-chlorid (zur Einführung eines Cl-Atoms) zu dem Diazoniumsalzen umgesetzt werden, die dann bei Temperaturen von 100°-140° thermisch zersetzt werden.

Die Verknüpfung eines aromatischen Kerns mit einem nicht aromatischen Kern oder zweier nicht aromatischer Kerne erhält man vorzugsweise durch Kondensation einer lithium- oder magnesiumorganischen Verbindung mit einem Keton bzw. einem Aldehyd oder Keton, falls zwischen den Kernen eine aliphatische Gruppe Z sein soll.

Die metallorganischen Verbindungen stellt man beispielsweise durch Metall-Halogenaustausch (z.B. nach Org. React. 6, 339-366 (1951)) zwischen der entsprechenden Halogen-Verbindung und einer lithiumorganischen Verbindung wie vorzugsweise tert-Butyllithium oder Lithium-Naphthalenid oder durch Umsatz mit Magnesiumspänen her.

Die Verknüpfung zweier aromatischer Ringe oder einer aliphatischen Gruppe Z mit einem aromatischen Ring erfolgt vorzugsweise durch Friedel-Crafts-Alkylierung oder Acylierung dadurch, daß man die entsprechenden Halogenverbindungen mit der entsprechenden aromatischen Verbindung unter Lewis-Säure-Katalyse usetzt. Geeignete Lewis-Säure sind z.B. $SnCl_4$, $ZnCl_2$ und besonders $AlCl_3$ und $TiCl_4$.

Weiterhin läßt sich die Verknüpfung zweier aromatischer Ringe durch die Ullmann-Reaktion (z.B. Synthesis 1974, 9) zwischen Aryljodiden mit Kupferjodid, vorzugsweise aber zwischen einer Aryl-Kupfer-Verbindung und einem Aryljodid, oder durch die Gomberg-Bachmann-Reaktion zwischen einem Aryl-Diazoniumsalz und der entsprechenden aromatischen Verbindung (z.B. Org. React. 2, 224 (1944)) durchführen. Die Darstellung der Tolane der Formel I (Z = -C≡C-) erfolgt z.B. durch Umsetzung der entsprechenden Arylhalogenide mit einem Acetylid in einem basischen Lösungsmittel unter Übergangsmetallkatalyse, bevorzugt können hier Palladium-Katalysatoren verwendet werden, insbesondere ein Gemisch aus Bis(Triphemylphosphin)palladium(II)chlorid und Kupferjodid in Piperidin als Lösungsmittel.

Darüberhinaus können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion sind Verbindungen entsprechend der Formel I, die aber an Stelle eines Cyclohexanringes einen Cyclohexenring oder Cyclohexanonring und/oder an Stelle einer $-CH_2CH_2-$Gruppe eine -CH=CH-Gruppe und/oder an Stelle einer $-CH_2-$Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa O° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit $NaBH_4$ oder Tributylzinnhydrid in Methanol hydriert werden.

Als Verdünnungsmittel eignen sich vorzugsweise Ether wie Diethylether, Dibutylether, Tetrahydrofuran, Dioxan, Amide wie Dimethylformamid, Hexamethylphosphorsäuretriamid, Kohlenwasserstoffe wie Hexan, Cyclohexan, Benzol, Toluol, Xylol, Halogenkohlenwasserstoffe wie Dichlormethan, Chloroform, Tetrachlorkohlenstoff, Tetrachlorethylen, Sulfoxide wie Dimethylsulfoxid, Sulfolan, Alkohole wie Methanol, Ethanol, Isopropanol und weitere organische Lösungsmittel wie Acetonitril und Nitromethan. Auch Wasser oder Gemische dieser Lösungsmittel untereinander und/oder mit Wasser sind für die vorstehende Umsetzung geeignet. Die Reaktionstemperaturen liegen zwischen -20 und +100°, vorzugsweise zwischen O und 50°. Bei diesen Temperaturen sind die Umsetzungen in der Regel nach 30 Minuten bis 24 Stunden beendet.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4′-Biscyclohexylbiphenyle, Phenyl- oder Cyclohexylpyrimidine, Phenyl- oder Cyclohexyldioxane, Phenyl- oder Cyclohexyldithiane, 1,2-Bis-phenylethane, 1,2-Biscyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R'\text{-}L\text{-}G\text{-}E\text{-}R'' \qquad II$$

worin L und E je ein carbo- oder heterocyclisches Ringsystem aus der aus 1,4-disubstituierten Benzol- und Cyclohexanringen, 4,4′-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G -CH=CH- -N(O)=N-
-CH=CY- -CH=N(O)-
-C≡C- $-CH_2-CH_2-$
-CO-O- $-CH_2-O-$
-CO-S- $-CH_2-S-$
-CH=N- -COO-Phe-COO-

oder eine C-C-Einfachbindung,

Y Halogen, vorzugsweise Chlor, oder -CN, und R′ und R″ Alkyl, Alkoxy, Alkanoyloxy oder Alkoxycarbonyloxy mit bis zu 18, vorzugsweise bis zu 8 Kohlenstoffatomen, oder einer dieser Reste auch CN, $NO_2$, $CF_3$, F, Cl oder Br bedeuten.

Bei den meisten dieser Verbindungen sind R′ und R″ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen flüssigkristallinen Phasen enthalten etwa 0,1 bis 99, vorzugsweise 10 bis 95 %, einer oder mehrerer Verbindungen der Formel I. Weiterhin bevorzugt sind flüssigkristalline Phasen, die 0,1 - 40, insbesondere 0,5 - 29 % einer oder mehrerer Verbindungen der Formel I enthalten.

Weiterhin bevorzugt sind erfindungsgemäße Dielektrika enthaltend 0,1 bis 40, vorzugsweise 0,5 bis 30 %, einer oder mehrerer Verbindungen der Formel I.

Verbindungen der Formel I mit optisch aktiver Flügelgruppe eignen sich als Komponenten nematischer flüssigkristalliner Phasen zur Vermeidung von reverse twist und zur Verbesserung der elastischen Konstanten.

Ferner sind die optisch aktiven Verbindungen der Formel I auch als Komponenten von chiral getilteten smektischen flüssigkristallinen Phasen geeignet.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 854, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozient; alle Temperaturangaben sind in Grad Celsius angegeben. "Übliche Aufarbeitung" bedeutet: man gibt gegebenenfalls Wasser hinzu, extrahiert mit Methylenchlorid, Ehter oder Toluol, trennt ab, trocknet die organische Phase, dampft ein und reinigt das Produkt durch Destillation unter reduziertem Druck oder Kristallisation und/oder Chromatographie.

Beispiel 1:

Ein Gemisch aus 11,6 g p-Pentylbenzoylchlorid, 11,1 g p-Hydroxyphenylschwefelpentafluorid und 100 ml Pyridin wird 20 Stunden bei Raumtemperatur gerührt. Nach Einengen und üblicher Aufarbeitung erhält man 4-(4-Pentylbenzoyloxy)phenylschwefelpentafluorid.

Analog werden dargestellt:

4-(4-Ethylbenzoyloxy)phenylschwefelpentafluorid
4-(4-Propylbenzoyloxy)phenylschwefelpentafluorid
4-(4-Butylbenzoyloxy)phenylschwefelpentafluorid
4-(4-Hexylbenzoyloxy)phenylschwefelpentafluorid
4-(4-Heptylbenzoyloxy)phenylschwefelpentafluorid
4-(4-Octylbenzoyloxy)phenylschwefelpentafluorid

4-(4-Methoxybenzoyloxy)phenylschwefelpentafluorid
4-(4-Ethoxybenzoyloxy)phenylschwefelpentafluorid
4-(4-Propoxybenzoyloxy)phenylschwefelpentafluorid
4-(4-Butoxybenzoyloxy)phenylschwefelpentafluorid
4-(4-Hexoxybenzoyloxy)phenylschwefelpentafluorid
4-(4-Heptoxybenzoyloxy)phenylschwefelpentafluorid
4-(4-Octoxybenzoyloxy)phenylschwefelpentafluorid

4-(4-(trans-4-Ethylcyclohexyl)benzoyloxy)phenylschwefelpentafluorid
4-(4-(trans-4-Propylcyclohexyl)benzoyloxy)phenylschwefelpentafluorid
4-(4-(trans-4-Butylcyclohexyl)benzoyloxy)phenylschwefelpentafluorid
4-(4-(trans-4-Hexylcyclohexyl)benzoyloxy)phenylschwefelpentafluorid
4-(4-(trans-4-Heptylcyclohexyl)benzoyloxy)phenylschwefelpentafluorid
4-(4-(trans-4-Octylcyclohexyl)benzoyloxy)phenylschwefelpentafluorid
4-(4-(trans-4-Methoxycyclohexyl)benzoyloxy)phenylschwefelpentafluorid
4-(4-(trans-4-Ethoxycyclohexyl)benzoyloxy)phenylschwefelpentafluorid
4-(4-(trans-4-Propoxycyclohexyl)benzoyloxy)phenylschwefelpentafluorid
4-(4-(trans-4-Butoxycyclohexyl)benzoyloxy)phenylschwefelpentafluorid
4-(4-(trans-4-Pentoxycyclohexyl)benzoyloxy)phenylschwefelpentafluorid
4-(4-(trans-4-Hexoxycyclohexyl)benzoyloxy)phenylschwefelpentafluorid
4-(4-(trans-4-Heptoxycyclohexyl)benzoyloxy)phenylschwefelpentafluorid
4-(4-(trans-4-Octoxycyclohexyl)benzoyloxy)phenylschwefelpentafluorid

4-(4-Ethylbenzoyloxy)-2-fluorphenylschwefelpentafluorid
4-(4-Propylbenzoyloxy)-2-fluorphenylschwefelpentafluorid
4-(4-Butylbenzoyloxy)-2-fluorphenylschwefelpentafluorid
4-(4-Pentylbenzoyloxy)-2-fluorphenylschwefelpentafluorid
4-(4-Hexylbenzoyloxy)-2-fluorphenylschwefelpentafluorid
4-(4-Heptylbenzoyloxy)-2-fluorphenylschwefelpentafluorid
4-(4-Octylbenzoyloxy)-2-fluorphenylschwefelpentafluorid
4-(4-Ethoxybenzoyloxy)-2-fluorphenylschwefelpentafluorid
4-(4-Propoxybenzoyloxy)-2-fluorphenylschwefelpentafluorid
4-(4-Butoxybenzoyloxy)-2-fluorphenylschwefelpentafluorid
4-(4-Pentoxybenzoyloxy)-2-fluorphenylschwefelpentafluorid
4-(4-Hexoxybenzoyloxy)-2-fluorphenylschwefelpentafluorid

4-(4-Ethylbenzoyloxy)-2-chlorphenylschwefelpentafluorid
4-(4-Propylbenzoyloxy)-2-chlorphenylschwefelpentafluorid
4-(4-Butylbenzoyloxy)-2-chlorphenylschwefelpentafluorid
4-(4-Pentylbenzoyloxy)-2-chlorphenylschwefelpentafluorid
4-(4-Hexylbenzoyloxy)-2-chlorphenylschwefelpentafluorid
4-(4-Heptylbenzoyloxy)-2-chlorphenylschwefelpentafluorid
4-(4-Octylbenzoyloxy)-2-chlorphenylschwefelpentafluorid
4-(4-Ethoxybenzoyloxy)-2-chlorphenylschwefelpentafluorid
4-(4-Propoxybenzoyloxy)-2-chlorphenylschwefelpentafluorid
4-(4-Butoxybenzoyloxy)-2-chlorphenylschwefelpentafluorid
4-(4-Pentoxybenzoyloxy)-2-chlorphenylschwefelpentafluorid
4-(4-Hexoxybenzoyloxy)-2-chlorphenylschwefelpentafluorid
4-(4-Heptoxybenzoyloxy)-2-chlorphenylschwefelpentafluorid
4-(4-Octoxybenzoyloxy)-2-chlorphenylschwefelpentafluorid

Beispiel 2

Ein Gemisch aus 9,1 g trans-4-Pentylcyclohexylcarbonsäure, 11,2 g p-Hydroxyphenylschwefelpentafluorid und 100 ml Methylenchlorid wird mit einem Gemisch aus 10,6 g Dicyclohexylcarbodiimid und 40 ml Methylenchlorid versetzt und 6 Stunden zum Sieden erhitzt. Nach üblicher Aufarbeitung erhält man 4-(trans-4-Pentylcyclohexylcarbonyloxy)phenyl-, schwefelpentafluorid.

Analog werden dargestellt:
4-(trans-4-Ethylcyclohexylcarbonyloxy)phenylschwefelpentafluorid
4-(trans-4-Propylcyclohexylcarbonyloxy)phenylschwefelpentafluorid
4-(trans-4-Butylcyclohexylcarbonyloxy)phenylschwefelpentafluorid
4-(trans-4-Hexylcyclohexylcarbonyloxy)phenylschwefelpentafluorid
4-(trans-4-Heptylcyclohexylcarbonyloxy)phenylschwefelpentafluorid
4-(trans-4-Octylcyclohexylcarbonyloxy)phenylschwefelpentafluorid

4-(trans-4-(trans-4-Ethylcyclohexyl)cyclohexylcarbonyloxy) phenylschwefelpentafluorid
4-(trans-4-(trans-4-Propylcyclohexyl)cyclohexylcarbonyloxy)phenylschwefelpentafluorid
4-(trans-4-(trans-4-Butylcyclohexyl)cyclohexylcarbonyloxy)phenylschwefelpentafluorid
4-(trans-4-(trans-4-Pentylcyclohexyl)cyclohexylcarbonyloxy)phenylschwefelpentafluorid
4-(trans-4-(trans-4-Hexylcyclohexyl)cyclohexylcarbonyloxy)phenylschwefelpentafluorid
4-(trans-4-(trans-4-Heptylcyclohexyl)cyclohexylcarbonyloxy)phenylschwefelpentafluorid
4-(trans-4-(trans-4-Octylcyclohexyl)cyclohexylcarbonyloxy)phenylschwefelpentafluorid

4-(trans-4-(4-Ethylphenyl)cyclohexylcarbonyloxy)phenylschwefelpentafluorid
4-(trans-4-(4-Propylphenyl)cyclohexylcarbonyloxy)phenylschwefelpentafluorid
4-(trans-4-(4-Butylphenyl)cyclohexylcarbonyloxy)phenylschwefelpentafluorid
4-(trans-4-(4-Pentylphenyl)cyclohexylcarbonyloxy)phenylschwefelpentafluorid
4-(trans-4-(4-Hexylphenyl)cyclohexylcarbonyloxy)phenylschwefelpentafluorid
4-(trans-4-(4-Heptylphenyl)cyclohexylcarbonyloxy)phenylschwefelpentafluorid
4-(trans-4-(4-Octylphenyl)cyclohexylcarbonyloxy)phenylschwefelpentafluorid
4-(trans-4-Ethylcyclhexylcarbonyloxy)-2-fluorphenylschwefelpentafluorid

4-(trans-4-Propylcyclohexylcarbonyloxy)-2-fluorphenylschwefelpentafluorid
4-(trans-4-Butylcyclohexylcarbonyloxy)-2-fluorphenylschwefelpentafluorid

4-(trans-4-Hexylcyclohexylcarbonyloxy)-2-fluorphenylschwefelpentafluorid
4-(trans-4-Heptylcyclohexylcarbonyloxy)-2-fluorphenylschwefelpentafluorid
4-(trans-4-Ethylcyclohexylcarbonyloxy)-2-chlorphenylschwefelpentafluorid
4-(trans-4-Propylcyclohexylcarbonyloxy)-2-chlorophenylschwefelpentafluorid
4-(trans-4-Butylcyclohexylcarbonyloxy)-2-chlorphenylschwefelpentafluorid
4-(trans-4-Hexylcyclohexylcarbonyloxy)-2-chlorphenylschwefelpentafluorid
4-(trans-4-Heptylcyclohexylcarbonyloxy)-2-chlorphenylschwefelpentafluorid
4-(trans-4-(4-Ethylphenyl)cyclohexylcarbonyloxy)-2-fluorphenylschwefelpentafluorid
4-(trans-4-(4-Propylphenyl)cyclohexylcarbonyloxy)-2-fluorphenylschwefelpentafluorid
4-(trans-4-(4-Butylphenyl)cyclohexylcarbonyloxy)-2-fluorphenylschwefelpentafluorid
4-(trans-4-(4-Pentylphenyl)cyclohexylcarbonyloxy)-2-fluorphenylschwefelpentafluorid
4-(trans-4-(4-Hexylphenyl)cyclohexylcarbonyloxy)-2-fluorphenylschwefelpentafluorid
4-(trans-4-(4-Heptylphenyl)cyclohexylcarbonyloxy)-2-fluorphenylschwefelpentafluorid
4-(trans-4-(4-Ethoxyphenyl)cyclohexylcarbonyloxy)-2-fluorphenylschwefelpentafluorid
4-(trans-4-(4-Propoxyphenyl)cyclohexylcarbonyloxy)-2-fluorphenylschwefelpentafluorid
4-(trans-4-(4-Butoxyphenyl)cyclohexylcarbonyloxy)-2-fluorphenylschwefelpentafluorid
4-(trans-4-(4-Pentoxyphenyl)cyclohexylcarbonyloxy)-2-fluorphenylschwefelpentafluorid
4-(trans-4-(4-Hexoxyphenyl)cyclohexylcarbonyloxy)-2-fluorphenylschwefelpentafluorid
4-(trans-4-(4-Heptoxyphenyl)cyclohexylcarbonyloxy)-2-fluorophenylschwefelpentafluorid
4-(trans-4-(4-Octoxyphenyl)cyclohexylcarbonyloxy)-2-fluorphenylschwefelpentafluorid

Beispiel 3

Ein Gemisch aus 4-Brommagnesiumphenylschwefelpentafluorid (hergestellt aus 5,6 g 4-Bromphenylschwefelpentafluorid, 0,5 g Magnesiumspänen und 0,05 g Jod) und 20 ml Tetrahydrofuran wird tröpfenweise mit einem Gemisch aus 2,9 g 4-Propylcyclohexanon und 10 ml Tetrahydrofuran versetzt. Nach Zugabe von 40 ml halbkonzentrierter Salzsäure, Abtrennen der festen Bestandteile, Abtrennen der wäßrigen Phase und Einengen, wird der verbleibende Rückstand in 40 ml Toluol gelöst, mit 5,0 g p-Toluolsulfonsäure versetzt und 3 Stunden zum Sieden erhitzt. Nach üblichem Aufarbeiten erhält man 4-(4-Propylcyclohex-1-enyl)phenylschwefelpentafluorid.

Analog werden hergestellt:
4-(4-Ethylcyclohex-1-enyl)phenylschwefelpentafluorid
4-(4-Butylcyclohex-1-enyl)phenylschwefelpentafluorid
4-(4-Pentylcyclohex-1-enyl)phenylschwefelpentafluorid
4-(4-Hexylcyclohex-1-enyl)phenylschwefelpentafluorid
4-(4-Heptylcyclohex-1-enyl)phenylschwefelpentafluorid
4-(4-Octylcyclohex-1-enyl)phenylschwefelpentafluorid

4-(4-(4-Ethylphenyl)cyclohex-1-enyl)phenylschwefelpentafluorid
4-(4-(4-Propylphenyl)cyclohex-1-enyl)phenylschwefelpentäfluorid
4-(4-(4-Butylphenyl)cyclohex-1-enyl)phenylschwefelpentafluorid
4-(4-(4-Pentylphenyl)cyclohex-1-enyl)phenylschwefelpentafluorid
4-(4-(4-Hexylphenyl)cyclohex-1-enyl)phenylschwefelpentafluorid
4-(4-(4-Heptylphenyl)cyclohex-1-enyl)phenylschwefelpentafluorid
4-(4-(4-Octylphenyl)cyclohex-1-enyl)phenylschwefelpentafluorid

4-(4-(trans-4-Ethylcyclohexyl)cyclohex-1-enyl)phenylschwefelpentafluorid
4-(4-(trans-4-Propylcyclohexyl)cyclohex-1-enyl)phenylschwefelpentafluorid
4-(4-(trans-4-Butylcyclohexyl)cyclohex-1-enyl)phenylschwefelpentafluorid
4-(4-(trans-4-Pentylcyclohexyl)cyclohex-1-enyl)phenylschwefelpentafluorid
4-(4-(trans-4-Hexylcyclohexyl)cyclohex-1-enyl)phenylschwefelpentafluorid
4-(4-(trans-4-Heptylcyclohexyl)cyclohex-1-enyl)phenylschwefelpentafluorid
4-(4-(trans-4-Octylcyclohexyl)cyclohex-1-enyl)phenylschwefelpentafluorid

Beispiel 4

Ein Gemisch aus 6,5 g 4-(4-Pentylcyclohex-1-enyl)phenylschwefelpentafluorid, 0,5 g Palladium/Aktivkohle (1 %) und 40 ml Toluol wird bei Raumtemperatur unter Normaldruck bis zur Sättigung hydriert. Nach Abdestillation des Lösungsmittels und üblicher Aufarbeitung erhält man 4-(trans-4-Pentylcyclohexyl)phenylschwefelpentafluorid.

Analog werden dargestellt:
4-(trans-4-Ethylcyclohexyl)-phenylschwefelpentafluorid
4-(trans-4-Butylcyclohexyl)-phenylschwefelpentafluorid
4-(trans-4-Pentylcyclohexyl)-phenylschwefelpentafluorid
4-(trans-4-Hexylcyclohexyl)-phenylschwefelpentafluorid
4-(trans-4-Heptylcyclohexyl)-phenylschwefelpentafluorid
4-(trans-4-Octylcyclohexyl)-phenylschwefelpentafluorid

4-(trans-4-(4-Ethylphenyl)cyclohexyl)-phenylschwefelpentafluorid
4-(trans-4-(4-Propylphenyl)cyclohexyl)-phenylschwefelpentafluorid
4-(trans-4-(4-Butylphenyl)cyclohexyl)-phenylschwefelpentafluorid
4-(trans-4-(4-Hexylphenyl)cyclohexyl)-phenylschwefelpentafluorid
4-(trans-4-(4-Heptylphenyl)cyclohexyl)-phenylschwefelpentafluorid
4-(trans-4-(4-Octylphenyl)cyclohexyl)-phenylschwefelpentafluorid

4-(trans-4-(trans-4-Ethylcyclohexyl)cyclohexyl)-phenylschwefelpentafluorid
4-(trans-4-(trans-4-Propylcyclohexyl)cyclohexyl)-phenylschwefelpentafluorid
4-(trans-4-(trans-4-Butylcyclohexyl)cyclohexyl)-phenylschwefelpentafluorid
4-(trans-4-(trans-4-Hexylcyclohexyl)cyclohexyl)-phenylschwefelpentafluorid
4-(trans-4-(trans-4-Heptylcyclohexyl)cyclohexyl)-phenylschwefelpentafluorid
4-(trans-4-(trans-4-Octylcyclohexyl)cyclohexyl)-phenylschwefelpentafluorid

Beispiel 5

Ein Gemisch der Zinkorganischen Verbindung, die aus 28,7 g 1-Brom-4-(trans-4-propylcyclohexyl)-cyclohexan und 7 g Zink hergestellt wurde, 13 g Nickel(II)chlorid und Tetrahydrofuran wird mit einem Gemisch aus 18,3 g 4-Bromphenylschwefelpentafluorid und 50 ml Tetrahydrofuran versetzt und 5 Stunden zum Sieden erhitzt. Nach üblicher Aufarbeitung erhält man 4-(trans-4-(trans-4-Propylcyclohexyl)cyclohexyl)phenylschwefelpentafluorid.

Analog werden dargestellt:

4-(trans-4-(trans-4-Ethylcyclohexyl)cyclohexyl)phenylschwefelpentafluorid
4-(trans-4-(trans-4-Butylcyclohexyl)cyclohexyl)phenylschwefelpentafluorid
4-(trans-4-(trans-4-Pentylcyclohexyl)cyclohexyl)phenylschwefelpentafluorid
4-(trans-4-(trans-4-Hexylcyclohexyl)cyclohexyl)phenylschwefelpentafluorid
4-(trans-4-(trans-4-Heptylcyclohexyl)cyclohexyl)phenylschwefelpentafluorid
4-(trans-4-(trans-4-Octylcyclohexyl)cyclohexyl)phenylschwefelpentafluorid

4(4-Ethylbicyclo[2.2.2]octyl)phenylschwefelpentafluorid
4(4-Propylbicyclo[2.2.2]octyl)phenylschwefelpentafluorid
4(4-Butylbicyclo[2.2.2]octyl)phenylschwefelpentafluorid
4(4-Pentylbicyclo[2.2.2]octyl)phenylschwefelpentafluorid
4(4-Hexylbicyclo[2.2.2]octyl)phenylschwefelpentafluorid
4(4-Heptylbicyclo[2.2.2]octyl)phenylschwefelpentafluorid
4(4-Octylbicyclo[2.2.2]octyl)phenylschwefelpentafluorid

4(4-Ethoxybicyclo[2.2.2]octyl)phenylschwefelpentafluorid
4(4-Propoxybicyclo[2.2.2]octyl)phenylschwefelpentafluorid
4(4-Butoxybicyclo[2.2.2]octyl)phenylschwefelpentafluorid
4(4-Pentoxybicyclo[2.2.2]octyl)phenylschwefelpentafluorid
4(4-Hexoxybicyclo[2.2.2]octyl)phenylschwefelpentafluorid
4(4-Heptoxybicyclo[2.2.2]octyl)phenylschwefelpentafluorid
4(4-Octoxybicyclo[2.2.2]octyl)phenylschwefelpentafluorid

## Beispiel 6

Ein Gemisch aus 4,4 g 4-Hydroxyphenylschwefelpentafluorid, 3,5 g Kaliumcarbonat und 20 ml Dimethylformamid wird mit einem Gemisch von 6,0 g trans-1-Jodmethyl-4-propylcyclohexan und 10 ml Dimethylformamid versetzt, 2 Stunden bei 100 °C gerührt. Nach Abtrennen fester Bestandteile und üblicher Aufarbeitung erhält man 4-(4-trans-Propylcyclohexylmethoxy)phenylschwefelpentafluorid.

Analog werden dargestellt:

4-(trans-4-Ethylcyclohexylmethoxy)phenylschwefelpentafluorid
4-(trans-4-Butylcyclohexylmethoxy)phenylschwefelpentafluorid
4-(trans-4-Pentylcyclohexylmethoxy)phenylschwefelpentafluorid
4-(trans-4-Hexylcyclohexylmethoxy)phenylschwefelpentafluorid
4-(trans-4-Heptylcyclohexylmethoxy)phenylschwefelpentafluorid
4-(trans-4-Octylcyclohexylmethoxy)phenylschwefelpentafluorid

4-(4-Ethylbicyclo[2.2.2]octylmethoxy)phenylschwefelpentafluorid
4-(4-Propylbicyclo[2.2.2]octylmethoxy)phenylschwefelpentafluorid
4-(4-Butylbicyclo[2.2.2]octylmethoxy)phenylschwefelpentafluorid
4-(4-Pentylbicyclo[2.2.2]octylmethoxy)phenylschwefelpentafluorid
4-(4-Hexylbicyclo[2.2.2]octylmethoxy)phenylschwefelpentafluorid
4-(4-Heptylbicyclo[2.2.2]octylmethoxy)phenylschwefelpentafluorid
4-(4-Octylbicyclo[2.2.2]octylmethoxy)phenylschwefelpentafluorid

4-(4-Ethylbenzyloxy)phenylschwefelpentafluorid
4-(4-Propylbenzyloxy)phenylschwefelpentafluorid
4-(4-Butylbenzyloxy)phenylschwefelpentafluorid
4-(4-Pentylbenzyloxy)phenylschwefelpentafluorid
4-(4-Hexylbenzyloxy)phenylschwefelpentafluorid
4-(4-Heptylbenzyloxy)phenylschwefelpentafluorid
4-(4-Octylbenzyloxy)phenylschwefelpentafluorid

4-(4-Ethoxybenzyloxy)phenylschwefelpentafluorid

4-(4-Propoxybenzyloxy)phenylschwefelpentafluorid
4-(4-Butoxybenzyloxy)phenylschwefelpentafluorid
4-(4-Pentoxybenzyloxy)phenylschwefelpentafluorid
4-(4-Hexoxybenyzloxy)phenylschwefelpentafluorid
4-(4-Heptoxybenzyloxy)phenylschwefelpentafluorid
4-(4-Octoxybenzyloxy)phenylschwefelpentafluorid

4-(4-(trans-4-Ethylcyclohexyl)benzyloxy)phenylschwefelpentafluorid
4-(4-(trans-4-Propylcyclohexyl)benzyloxy)phenylschwefelpentafluorid
4-(4-(trans-4-Butylcyclohexyl)benzyloxy)phenylschwefelpentafluorid
4-(4-(trans-4-Pentylcyclohexyl)benzyloxy)phenylschwefelpentafluorid
4-(4-(trans-4-Hexylcyclohexyl)benzyloxy)phenylschwefelpentafluorid
4-(4-(trans-4-Heptylcyclohexyl)benzyloxy)phenylschwefelpentafluorid
4-(4-(trans-4-Octylcyclohexyl)benzyloxy)phenylschwefelpentafluorid

Beispiel 7

Ein Gemisch aus 8,5 g 4-Pentoxyphenylacetylen, 20 ml Piperidin, 420 mg Triphenylphosphinpalladium(II)-chlorid und 30 mg Kupferjodid wird mit 8,5 g p-Bromphenylschwefelpentafluorid und 20 ml Piperidin versetzt und 20 Stunden erhitzt. Nach Einengen und üblichem Aufarbeiten erhält man 4-(2-(4-Pentoxyphenyl)ethinyl)phenylschwefelpentafluorid.

Analog werden dargestellt:
4-(2-(4-Ethoxyphenyl)ethinyl)phenylschwefelpentafluorid
4-(2-(4-Propoxyphenyl)ethinyl)phenylschwefelpentafluorid
4-(2-(4-Butoxyphenyl)ethinyl)phenylschwefelpentafluorid
4-(2-(4-Hexoxyphenyl)ethinyl)phenylschwefelpentafluorid
4-(2-(4-Heptoxyphenyl)ethinyl)phenylschwefelpentafluorid
4-(2-(4-Octoxyphenyl)ethinyl)phenylschwefelpentafluorid
4-(2-(4-Ethylphenyl)ethinyl)phenylschwefelpentafluorid
4-(2-(4-Propylphenyl)ethinyl)phenylschwefelpentafluorid
4-(2-(4-Butylphenyl)ethinyl)phenylschwefelpentafluorid
4-(2-(4-Pentylphenyl)ethinyl)phenylschwefelpentafluorid
4-(2-(4-Hexylphenyl)ethinyl)phenylschwefelpentafluorid
4-(2-(4-Heptylphenyl)ethinyl)phenylschwefelpentafluorid
4-(2-(4-Octylphenyl)ethinyl)phenylschwefelpentafluorid

4-(2-(trans-4-Ethylcyclohexyl)ethinyl)phenylschwefelpentafluorid
4-(2-(trans-4-Propylcyclohexyl)ethinyl)phenylschwefelpentafluorid
4-(2-(trans-4-Butylcyclohexyl)ethinyl)phenylschwefelpentafluorid
4-(2-(trans-4-Pentylcyclohexyl)ethinyl)phenylschwefelpentafluorid
4-(2-(trans-4-Hexylcyclohexyl)ethinyl)phenylschwefelpentafluorid
4-(2-(trans-4-Heptylcyclohexyl)ethinyl)phenylschwefelpentafluorid
4-(2-(trans-4-Octylcyclohexyl)ethinyl)phenylschwefelpentafluorid
4-(2-(trans-4-Ethoxycyclohexyl)ethinyl)phenylschwefelpentafluorid
4-(2-(trans-4-Propoxycyclohexyl)ethinyl)phenylschwefelpentafluorid
4-(2-(trans-4-Butoxycyclohexyl)ethinyl)phenylschwefelpentafluorid
4-(2-(trans-4-Hexoxycyclohexyl)ethinyl)phenylschwefelpentafluorid
4-(2-(trans-4-Heptoxycyclohexyl)ethinyl)phenylschwefelpentafluorid
4-(2-(trans-4-Octoxycyclohexyl)ethinyl)phenylschwefelpentafluorid

Beispiel 8

Ein Gemisch aus 11,4 g 4-(2-(4-trans-Pentylcyclohexyl)ethinyl)phenylschwefelpentafluorid (dargestellt nach Beispiel 7), 50 ml Toluol und 10 mg Palladium/Aktivkohle wird bei Raumtemperatur unter Normaldruck hydriert. Nach Abfiltrieren fester Bestandteile, Einengen und üblicher Aufarbeitung erhält man 4-(2-(trans-4-Pentylcyclohexyl)ethyl)phenylschwefelpentafluorid.

Analog werden dargestellt:
4-(2-(trans-4-Ethylcyclohexyl)ethyl)phenylschwefelpentafluorid

4-(2-(trans-4-Propylcyclohexyl)ethyl)phenylschwefelpentafluorid
4-(2-(trans-4-Butylcyclohexyl)ethyl)phenylschwefelpentafluorid
4-(2-(trans-4-Hexylylcyclohexyl)ethyl)phenylschwefelpentafluorid
4-(2-(trans-4-Heptylcyclohexyl)ethyl)phenylschwefelpentafluorid
4-(2-(trans-4-Octylcyclohexyl)ethyl)phenylschwefelpentafluorid
4-(2-(trans-4-Ethoxycyclohexyl)ethyl)phenylschwefelpentafluorid
4-(2-(trans-4-Propoxycyclohexyl)ethyl)phenylschwefelpentafluorid
4-(2-(trans-4-Butoxycyclohexyl)ethyl)phenylschwefelpentafluorid
4-(2-(trans-4-Hexyloxycyclohexyl)ethyl)phenylschwefelpentafluorid
4-(2-(trans-4-Heptoxycyclohexyl)ethyl)phenylschwefelpentafluorid
4-(2-(trans-4-Octoxycyclohexyl)ethyl)phenylschwefelpentafluorid

4-(2-(4-Ethylphenyl)ethyl)phenylschwefelpentafluorid
4-(2-(4-Propylphenyl)ethyl)phenylschwefelpentafluorid
4-(2-(4-Butylphenyl)ethyl)phenylschwefelpentafluorid
4-(2-(4-Pentylphenyl)ethyl)phenylschwefelpentafluorid
4-(2-(4-Hexylylphenyl)ethyl)phenylschwefelpentafluorid
4-(2-(4-Heptylphenyl)ethyl)phenylschwefelpentafluorid
4-(2-(4-Octylphenyl)ethyl)phenylschwefelpentafluorid
4-(2-(4-Ethoxyphenyl)ethyl)phenylschwefelpentafluorid
4-(2-(4-Propoxyphenyl)ethyl)phenylschwefelpentafluorid
4-(2-(4-Butoxyphenyl)ethyl)phenylschwefelpentafluorid
4-(2-(4-Pentoxyphenyl)ethyl)phenylschwefelpentafluorid
4-(2-(4-Hexyloxyphenyl)ethyl)phenylschwefelpentafluorid
4-(2-(4-Heptoxyphenyl)ethyl)phenylschwefelpentafluorid
4-(2-(4-Octoxyphenyl)ethyl)phenylschwefelpentafluorid

Beispiel A

Man stellt eine Flüssigkristallmischung her bestehend aus

10 % p-Ethylbenzoesäure-(p-cyanphenylester)
11 % p-Butylbenzoesäure-(p-cyanphenylester)
9 % trans-4-Propylcyclohexancarbonsäure-(p-ethoxyphenylester)
24 % trans-4-Propylcyclohexancarbonsäure-(p-butoxyphenylester)
19 % trans-4-Butylcyclohexancarbonsäure-(p-ethoxyphenylester)
21 % trans-4-Pentylcyclohexancarbonsäure-(p-methoxyphenylester) und
6 % 4-(4-Pentylbenzoyloxy)-phenylschwefelpentafluorid.

## Patentansprüche

**1.** Arylschwefelpentafluoride der Formel I,

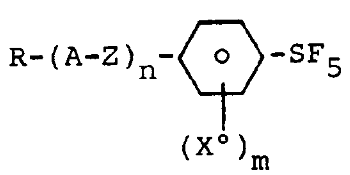

I

worin

R H, Y oder ein unsubstituierter oder durch mindestens eine Gruppe Y substituierter Alkyl- oder Alkenylrest mit jeweils 1-15 C-Atomen, worin auch eine oder mehrere nicht benachbarte $CH_2$-Gruppen durch O- und/oder S-Atome und/oder durch -E-, -CO-, -O-CO-, -CO-O-, -O-CO-O-, -S-CO- und/oder -CO-S- Gruppen ersetzt sein können,

E C≡C,

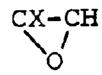 oder 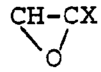,

X° jeweils unabhängig voneinander Y oder $CH_3$,
X jeweils unabhängig voneinander X° oder H,
Y CN, $N_3$, NCO, NCS, Fluor oder Chlor
A jeweils unabhängig voneinander

a) 1,4-Cyclohexylenrest,
worin eine oder zwei nicht benachbarte $CH_2$- Gruppen durch O-Atome ersetzt sein können,
b) 1,4-Phenylenrest,
worin ein oder mehrere CH-Gruppen durch N ersetzt sein können,
c) Rest aus der Gruppe 1,4-Cyclohexenylen, Piperidin-1,4-diyl, Bicyclo(2,2,2)octylen, Naphthalin-2,6-diyl, Decahydronaphthalin-2,6-diyl oder 1,2,3,4-Tetrahydronaphthalin, wobei die Reste a) und b) ein- oder zweifach durch X° substituiert sein können,

Z jeweils unabhängig voneinander -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -CH=N-, -N=CH-, -NO=N-, -N=NO- oder eine Einfachbindung,
m 0, 1 oder 2, und
n 1, 2 oder 3 bedeutet.

2. Verfahren zur Herstellung von Arylschwefelpentafluoriden der Formel I, dadurch gekennzeichnet, daß man p-Lithium- oder p-Brommagnesiumphenylschwefelpentafluoride mit entsprechenden Elektrophilen umsetzt,
oder daß man entsprechende 1-Alkine mit p-Bromphenylschwefelpentafluorid unter Übergansmetall-Katalyse koppelt,
oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,
oder daß man zur Herstellung von Estern der Formel I (worin Z -CO-O- oder -O-CO- bedeutet und/oder R eine Carboxylgruppe enthält) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Ethern der Formel I (worin R eine Alkoxygruppe bedeutet und/oder Z eine $-OCH_2-$ oder $-CH_2O$-Gruppe ist) eine entsprechende Hydroxyverbindung verethert.

3. Flüssigkristalline Phase mit mindestens zwei Komponenten, dadurch gekennzeichnet, daß sie mindestens eine Verbindung der Formel I enthält.

4. Flüssigkristallanzeigeelement, dadurch gekennzeichnet, daß es eine flüssigkristalline Phase nach Anspruch 3 enthält.

5. Elektrooptisches Anzeigeelement, dadurch gekennzeichnet, daß es als Dielektrikum eine Phase nach Anspruch 3 enthält.

## Claims

1. Aryl-sulfur pentafluorides of the formula I

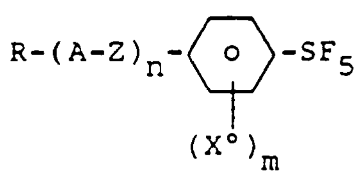

wherein
R is H, Y or an alkyl or alkenyl radical each of which has 1-15 C atoms and is unsubstituted or substituted by at least one group Y and in which one or more non-adjacent $CH_2$ groups can also be

replaced by O and/or S atoms and/or by -E-, -CO-, -O-CO-, -CO-O-, -O-CO-O-. -S-CO- and/or -CO-S-groups,

E is C≡C,

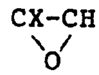 or 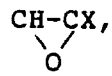,

the X°s independently of one another are each Y or $CH_3$,
the Xs independently of one another are each X° or H,
Y is CN, $N_3$, NCO, NCS, fluorine or chlorine,
the As independently of one another are each
a) a 1,4-cyclohexylene radical,
wherein one or two non-adjacent $CH_2$ groups can be replaced by O atoms,
b) a 1,4-phenylene radical,
wherein one or more CH groups can be replaced by N, or
c) a radical belonging to the group comprising 1,4-cyclohexenylene, piperidine-1,4-diyl, bicyclo-(2.2.2)octylene,naphthalene-2,6-diyl,decahydronaphthalene-2,6-diyl or 1,2,3,4,-tetrahydronaphthalene, it being possible for the radicals a) and b) to be monosubstituted or disubstituted by X°,
the Zs independently of one anther are each -CO-O-, -O-CO-, $-CH_2CH_2-$, $-CHCN-CH_2-$, $-CH_2-CHCN-$, -CH=CH- -C≡C-, $-OCH_2-$, $-CH_2O-$, -CH=N-, -N=CH-, -NO=N-, N=NO or a single bond,
m is 0, 1 or 2 and
n is 1, 2 or 3.

**2.** Process for the preparation of aryl-sulfur pentafluorides of the formula I, characterized in that p-lithium-phenyl or p-bromomagnesium-phenyl-sulfur pentafluorides are reacted with appropriate electrophiles, or corresponding 1-alkynes are coupled with p-bromophenylsulfur pentafluorides with transition metal catalysis, or a compound which otherwise corresponds to the formula 1, but contains one or more reducible groups and/or C-C bonds instead of H atoms, is treated with a reducing agent, or, in order to prepare esters of the formula I (wherein Z is -CO-O- or -O-CO- and/or R contains a carboxyl group), a corresponding carboxylic acid or one of its reactive derivatives is reacted with a corresponding alcohol or one of its reactive derivatives, or, in order to prepare ethers of the formula I (wherein R is an alkoxy group and/or Z is an $-OCH_2-$ or $-CH_2O-$ group), a corresponding hydroxy compound is etherified.

**3.** Liquid-crystal phase having at least two components, characterized in that it contains at least one compound of the formula I.

**4.** Liquid-crystal display element characterized in that it contains a liquid-crystal phase according to Claim 3.

**5.** Electrooptical display element characterized in that it contains a phase according to Claim 3 as the dielectric.

## Revendications

**1.** Arylpentafluorures de soufre de formule I

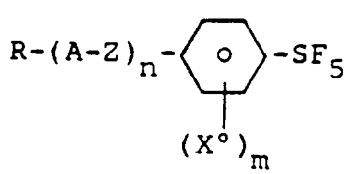

I

dans laquelle

R représente H, Y ou un reste alkyle ou alkylène non substitué ou substitué par au moins un groupe Y avec respectivement 1-15 atomes de C, dans lequel aussi un ou plusieurs groupes $CH_2$ non

voisins peuvent être remplacés par des atomes de O et/ou de S et/ou par des groupes -E-, -CO-, -O-CO-, -CO-O-, -O-CO-O-, -S-CO- et/ou -CO-S-,

E représente C≡C,

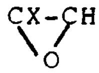 ou 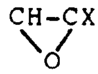,

X° représente indépendamment de l'autre Y ou $CH_3$,

X représente indépendamment de l'autre X° ou H,

Y représente CN, $N_3$, NCO, NCS, fluor ou chlore,

A représente indépendamment

a) un reste 1,4-cyclohexylène, dans lequel un ou plusieurs groupes $CH_2$- non voisins peuvent être remplacés par des atomes de O,

b) un reste 1,4-phénylène, dans lequel un ou plusieurs groupes CH peuvent être remplacés par N,

c) un reste du groupe des 1,4-cyclohexénylène, pipéridin-1,4-diyle, bicyclo(2,2,2)octylène, naphtalèn-2,6-diyle, décahydronaphtalène-2,6-diyle ou 1,2,3,4-tétrahydronaphtalène, les restes a) et b) pouvant être substitués une ou deux fois par X°,

Z représente indépendamment -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -CH=N-, -N=CH-, -NO=N, -N=NO- ou une liaison simple,

m est 0, 1 ou 2, et

n est 1, 2 ou 3.

**2.** Procédé de préparation d'arylpentafluorure de soufre de formule I, caractérisé en ce qu'on fait réagir du p-lithium- ou p-bromomagnésiumphénylpentafluorure de soufre avec des composés électrophiles correspondants, ou en ce qu'on additionne le 1-alcyne correspondant au p-bromophénylpentafluorure de soufre à l'aide de la catalyse par les métaux de transition, ou qu'on traite avec un agent réducteur un composé, gui sinon correspond à la formule I, mais possède à la place d'atomes de H, un ou plusieurs groupes qu'on peut réduire et/ou des liaisons C-C,

ou que pour préparer des esters de formule I (dans laquelle Z représente -CO-O- ou -O-CO- et/ou R contient un groupe carboxyle) on fait réagir un acide carboxylique correspondant ou un de ses dérivés pouvant réagir avec un alcool approprié ou un de ses dérivés pouvant réagir,

ou que pour préparer des éthers de formule I (dans laquelle R représente un groupe alcoxy et/ou Z est un groupe $-OCH_2-$ ou $-CH_2O-$) on éthérifie un composé hydroxy approprié.

**3.** phase de cristaux liquides avec au moins deux composants, caractérisée en ce qu'elle contient au moins un composé de formule I.

**4.** Elément d'affichage à cristaux liquides, caractérisé en ce qu'il contient une phase de cristaux liquides selon la revendication 3.

**5.** Elément d'affichage électrooptique, caractérisé en ce qu'il contient comme diélectrique une phase selon la revendication 3.